# EUROPEAN PATENT APPLICATION

(11) **EP 4 406 955 A1**
(43) Date of publication of application: **31.07.2024**
(21) Application number: 22872116.3
(22) Date of filing: 23.09.2022
(51) Int. Cl.: C07D 473/32, A61K 31/52, A61P 35/00

(54) **CRYSTAL FORM OF IMIDAZOLINONE DERIVATIVE**

(30) Priority: 23.09.2021 CN 202111112867
(71) Applicant: Chengdu Baiyu Pharmaceutical Co., Ltd., Chengdu, Sichuan 611130 (CN)
(72) Inventor: XU, Xuezhen, Chengdu, Sichuan 611130 (CN); LEI, Feiquan, Chengdu, Sichuan 611130 (CN); HE, Lvxue, Chengdu, Sichuan 611130 (CN); WEI, Yonggang, Chengdu, Sichuan 611130 (CN); SUN, Yi, Chengdu, Sichuan 611130 (CN)
(74) Representative: Patentanwälte Olbricht Buchhold Keulertz
(86) International application number: PCT/CN2022/120890
(87) International publication number: WO 2023/046072

(57) **Abstract**

The present invention relates to a crystal form of an imidazolinone derivative. The present invention further relates to a crystal form of a substituted imidazolinone derivative and a pharmaceutical composition thereof, and a preparation method therefor and the use thereof in the preparation of a DNA-PK inhibitor. Specifically, the present invention relates to crystal forms I-VI of the compound as represented by formula (A) and a pharmaceutical composition thereof, and a preparation method therefor and the use thereof in the preparation of a DNA-PK inhibitor.

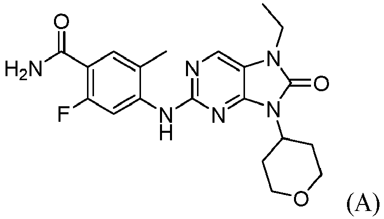

## Description

### Field of Technology

The present invention relates to a crystalline form of an imidazolinone derivative, or a hydrate or solvate thereof, as well as a preparation method or a pharmaceutical composition thereof and its use in the field of DNA-PK inhibitor preparation.

### Background Art

DNA-dependent protein kinase (DNA-PK) is a DNA-PK enzyme complex composed of Ku70/Ku80 heterodimers and DNA-dependent protein kinase catalytic subunits (DNA-PKcs). The enzyme complex needs the participation of DNA in order to be activated for its appropriate function (George et al., 2019). As a serine/threonine protein kinase, DNA-PK is a member of the PIKK (phosphatidylinositol 3-kinase-related kinase) family, which not only plays an important role in the repair of intracellular DNA double-strand breaks (DSBs) and cellular DNA recombination or antibody DNA rearrangement (V(D)J recombination), but also participates in physiological processes such as chromosome modification, transcriptional regulation, and telomere maintenance.

Combining DNA-PK inhibitors with DNA damage-inducing antitumor therapies (e.g., IR, chemotherapeutic reagents, etc.) can improve the therapeutic efficacy. The use of DNA-PK inhibitors may somehow interfere with the DNA repair function of normal cells, but there are various complementary pathways for DNA in normal cells. Tumor cells are more sensitive to DNA-PK inhibitors due to the high pressure from DNA replication and the lack of effective DNA repair mechanism, and the inhibition of DNA-PK activities in tumor cells can enhance the killing effect of other anti-tumor therapies in tumor cells.

Patent Application PCT/CN2021/087912 describes a novel DNA-PK inhibitor with the structure represented by formula (A). It has a good inhibitory effect on DNA-PK activity and has the potential in the preparation of anti-tumor drugs.

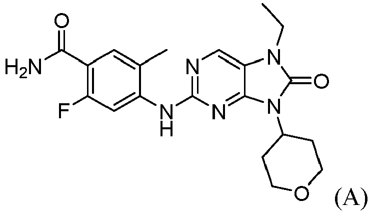

### Summary of the Invention

The present invention provides a crystalline form of 4-((7-ethyl-8-oxo-9-(tetrahydro-2H-pyran-4-yl)-8,9-dihydro-7H-purin-2-yl)amino)-2-fluoro-5-methylbenzamide (Compound A) which has the following chemical structure:

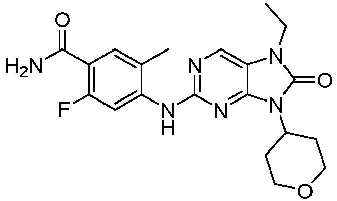

The crystalline form of the present invention exhibits at least one of the following advantages: good solubility, high stability, ease in handling, processing and purification, improved oral bioavailability of the drug, prolonged storage period of the drug, and ease in the manufacture of various dosage forms.

The crystalline form of the present invention exhibits pharmaceutical advantages over an amorphous form of Compound A. In particular, the crystalline form enhances chemical and physical stability and is more favorable in the preparation of solid pharmaceutical dosage forms comprising pharmacologically active ingredients.

The crystalline form of the present invention is present in an amount of from about 5 wt% to about 100 wt% of the active pharmaceutical ingredient (API). In certain embodiments, the crystalline form of the present invention is present in an amount of from about 10 wt% to about 100 wt% of the API. In certain embodiments, the crystalline form of the present invention is present in an amount of from about 15 wt% to about 100 wt% of the API. In certain embodiments, the crystalline form of the present invention is present in an amount of from about 20 wt% to about 100 wt% of the API. In certain embodiments, the crystalline form of the present invention is present in an amount of from about 25 wt% to about 100 wt% of the API. In certain embodiments, the crystalline form of the present invention is present in an amount of from about 30 wt% to about 100 wt% of the API. In certain embodiments, the crystalline form of the present invention is present in an amount of from about 35 wt% to about 100 wt% of the API. In certain embodiments, the crystalline form of the present invention is present in an amount of from about 40 wt% to about 100 wt% of the API. In certain embodiments, the crystalline form of the present invention is present in an amount of from about 45 wt% to about 100 wt% of the API. In certain embodiments, the crystalline form of the present invention is present in an amount of from about 50 wt% to about 100 wt% of the API. In certain embodiments, the crystalline form of the present invention is present in an amount of from about 55 wt% to about 100 wt% of the API. In certain embodiments, the crystalline form of the present invention is present in an amount of from about 60 wt% to about 100 wt% of the API. In certain embodiments, the crystalline form of the present invention is present in an amount of from about 65 wt% to about 100 wt% of the API. In certain embodiments, the crystalline form of the present invention is present in an amount of from about 70 wt% to about 100 wt% of the API. In certain embodiments, the crystalline form of the present invention is present in an amount of from about 75 wt% to about 100 wt% of the API. In certain embodiments, the crystalline form of the present invention is present in an amount of from about 80 wt% to about 100 wt% of the API. In certain embodiments, the crystalline form of the present invention is present in an amount of from about 85 wt% to about 100 wt% of the API. In certain embodiments, the crystalline form of the present invention is present in an amount of from about 90 wt% to about 100 wt% of the API. In certain embodiments, the crystalline form of the present invention is present in an amount of from about 95 wt% to about 100 wt% of the API. In certain embodiments, the crystalline form of the present invention is present in an amount of from about 98 wt% to about 100 wt% of the API. In certain embodiments, the crystalline form of the present invention is present in an amount of from about 99 wt% to about 100 wt% of the API. In certain embodiments, substantially all the API is the crystalline form of the present invention, i.e., the API is substantially a phase-pure crystal.

Compound A of the present invention is in an amorphous form, unless otherwise specified.

In an embodiment of the present invention, the crystalline form is anhydrous Compound A (crystalline form I), and has characteristic diffraction peaks at 2θ positions of 9.859°±0.3°, 14.759°±0.3°, 19.679°±0.3°, and 19.961°±0.3° in an X-ray powder diffraction pattern obtained by Cu-Kα radiation.

Herein, in the X-ray powder diffraction pattern of the crystalline form I, characteristic diffraction peaks are also present at 2θ positions of 4.979°±0.2°, 15.759°±0.2°, 19.339°±0.2°, 22.481°±0.2°, and 24.641°±0.2°.

Further, the crystalline form I further has characteristic diffraction peaks at 2θ positions of 12.120°±0.2°, 18.892°±9.2°, 21.822°±9.2° in the X-ray powder diffraction pattern.

Furthermore, the crystalline form I further has characteristic diffraction peaks at 2θ positions of 11.483°±0.2°, 20.422°±0.2°, 21.379°±0.2°, 22.142°±0.2°, 25.939°±0.2°, 29.180°±0.2°, 31.941°±9.2° in the X-ray powder diffraction pattern.

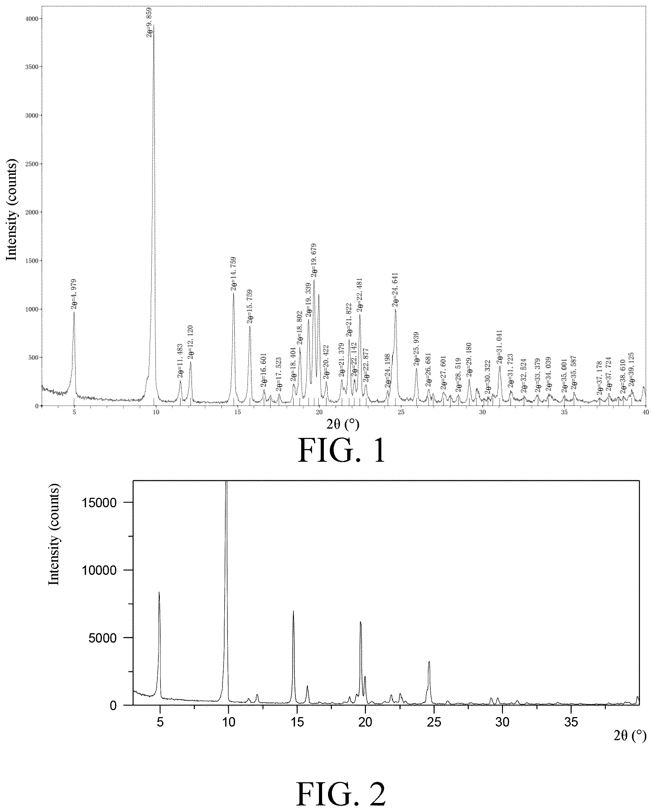

Even further, the crystalline form I has an X-ray powder diffraction pattern substantially as shown in Fig. 1 or 2.

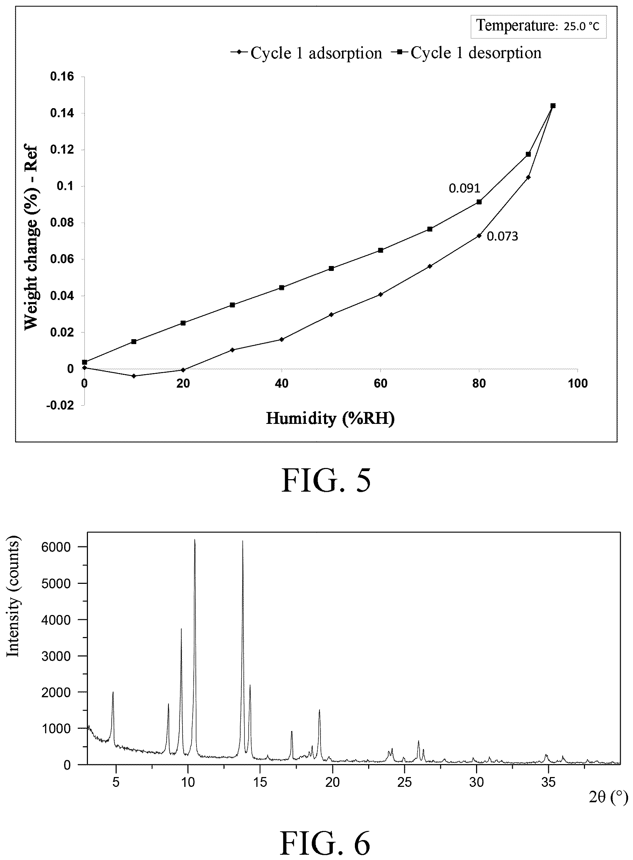

In an embodiment of the present invention, the crystalline form is crystalline form II, which has an X-ray powder diffraction pattern substantially as shown in Fig. 6.

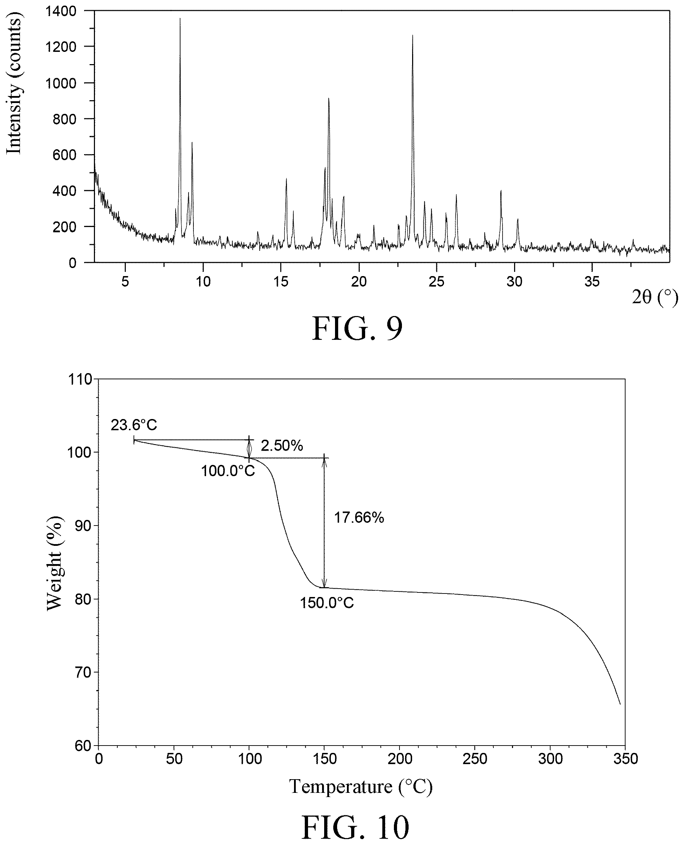

In an embodiment of the present invention, the crystalline form is crystalline form III, which has an X-ray powder diffraction pattern substantially as shown in Fig. 9.

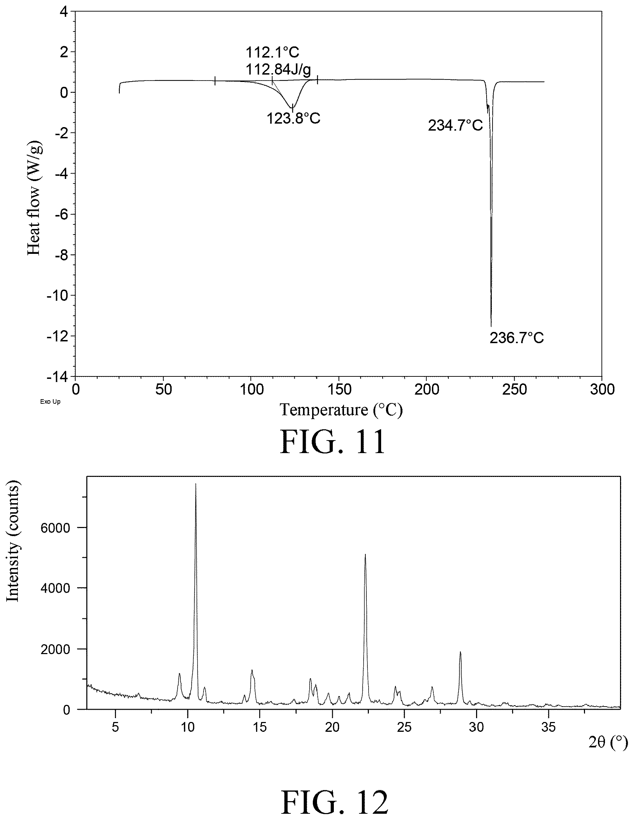

In an embodiment of the present invention, the crystalline form is crystalline form IV, which has an X-ray powder diffraction pattern substantially as shown in Fig. 12.

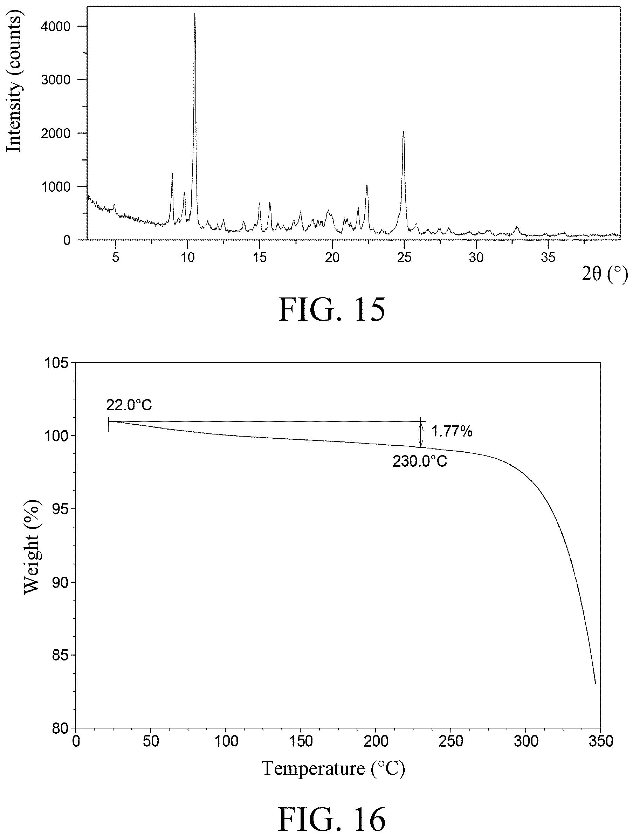

In an embodiment of the present invention, the crystalline form is crystalline form V, which has an X-ray powder diffraction pattern substantially as shown in Fig. 15.

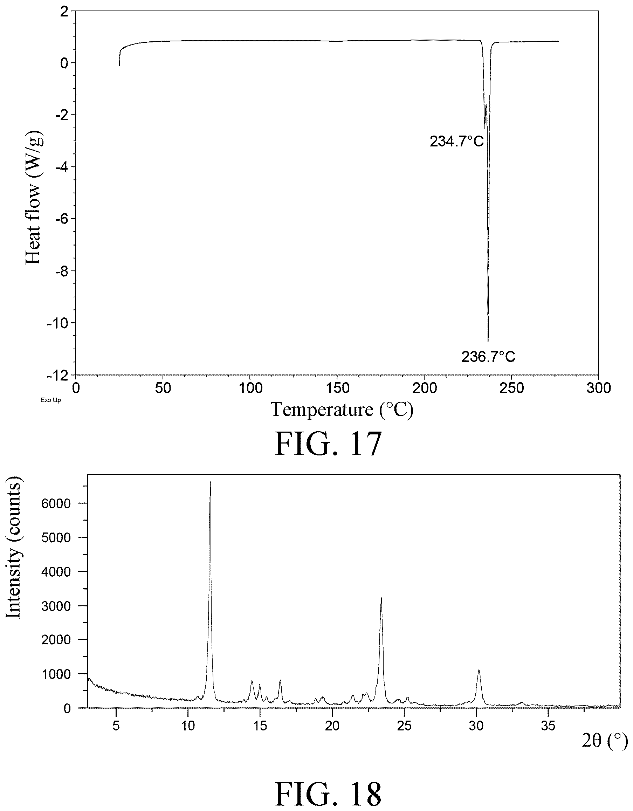

In an embodiment of the present invention, the crystalline form is crystalline form VI, which has an X-ray powder diffraction pattern substantially as shown in Fig. 18.

The present invention also relates to a method for preparing the crystalline form I, which is Method I or Method II:
Method I: dissolving Compound A in a solvent, heating to reflux till the solid is dissolved, and then naturally cooling down for crystallization, followed by filtering and drying, to obtain the crystalline form I;
Method II: dissolving Compound A in a solvent, heating to 75-85°C till the solid is dissolved, then cooling down to 55-65°C for crystallization, and further cooling down to 15-25°C for crystallization, followed by filtering and drying, to obtain the crystalline form I;
wherein the solvent is selected from an alcohol-based solvent or a mixed solvent of an alcohol-based solvent and water.

The present invention also relates to a pharmaceutical composition comprising a therapeutically effective amount of a compound in the crystalline form according to the present invention, and one or more of a pharmaceutically acceptable carrier or excipient.

The crystalline forms of the present invention as active pharmaceutical ingredients, or a pharmaceutical composition in which the crystalline forms is used as active ingredients may be for use in the preparation of a DNA-PK inhibitor.

Herein, the DNA-PK inhibitor is for use in the preparation of a medicament for the treatment and prevention of cancer.

The X-ray powder diffraction patterns disclosed herein and those which are substantially the same are within the scope of the present invention.

Unless stated to the contrary, the terms used in the specification and claims have the following meanings.

An "effective dose" means an amount, which is sought for, of a compound which results in a physiological or medical response in a tissue, system, or subject, including an amount of a compound which is sufficient to prevent the onset of, or reduce to some degree, one or more of the symptoms of the disease or condition to be treated, when administered to the subject.

"IC₅₀" refers to the half inhibitory concentration, meaning the concentration at which half of the maximum inhibitory effect is reached.

The crystalline structure according to the present invention may be analyzed using various analytical techniques known to those of ordinary skill in the art, including, but not limited to, X-ray powder diffraction (XRD).

It will be appreciated that the values described and sought for protection by the present invention are approximate values. Variations within the values may be attributed to the calibration of the equipment, error of the equipment, purity of the crystal, crystal size, sample size, and other factors.

It will be appreciated that the crystalline forms according to the present invention are not limited to characteristic patterns identical to those illustrated in the accompanying drawings disclosed herein, such as XRD. Any crystalline forms having a characteristic pattern that is substantially or essentially identical to those patterns illustrated in the accompanying drawings falls within the scope of the present invention.

Without departing from the scope and spirit of the present invention, various modification and alteration of the present invention will be apparent to those skilled in the art upon consideration of the disclosure of the specification and procedures in the examples of the present invention.

### Brief Description of the Drawings

Fig. 1 is an X-ray powder diffraction pattern obtained with a Cu-Kα radiation of the crystalline form I of compound A prepared in Example 2.
Fig. 2 is an X-ray powder diffraction pattern of the crystalline form I of compound A prepared in Example 3.
Fig. 3 is a TGA diagram of the crystalline form I of compound A prepared in Example 3.
Fig. 4 is a DSC diagram of the crystalline form I of compound A prepared in Example 3.
Fig. 5 is a DVS diagram of the crystalline form I of compound A prepared in Example 3.
Fig. 6 is an X-ray powder diffraction pattern of crystalline form II.
Fig. 7 is a TGA diagram of the crystalline form II.
Fig. 8 is a DSC diagram of the crystalline form II.
Fig. 9 is an X-ray powder diffraction pattern of crystalline form III.
Fig. 10 is a TGA diagram of crystalline form III.
Fig. 11 is a DSC diagram of crystalline form III.
Fig. 12 is an X-ray powder diffraction pattern of crystalline form IV.
Fig. 13 is a TGA diagram of crystalline form IV.
Fig. 14 is a DSC diagram of crystalline form IV.
Fig. 15 is an X-ray powder diffraction pattern of crystalline form V.
Fig. 16 is a TGA diagram of crystalline form V.
Fig. 17 is a DSC diagram of crystalline form V.
Fig. 18 is an X-ray powder diffraction pattern of crystalline form VI.
Fig. 19 is a TGA diagram of crystalline form VI.
Fig. 20 is a DSC diagram of crystalline form VI.

### Detailed Description of the Invention

The following detailed description of the implementation process and the beneficial effects produced by the present invention by means of specific examples is intended to help the reader better understand the essence and characteristics of the present invention, and not as a limitation on the implementable scope of the present application.

Unless otherwise specified in the examples, a solution refers to an aqueous solution.

Unless otherwise specified, the experimental conditions for crystallization are generally at room temperature (20-30°C, 30-70% RH), and a solvent ratio refers to a volume ratio.

### Intermediate 1

### 4-amino-2-fluoro-5-methylbenzamide (Intermediate 1)

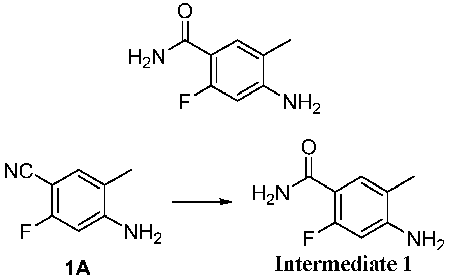

4-amino-2-fluoro-5-methylbenzonitrile 1A (300 mg, 2 mmol) and potassium carbonate (41.4 mg, 0.3 mmol) were dissolved in 1 mL of dimethylsulfoxide and put in an ice bath, into which 300 µL of hydrogen peroxide was added, followed by gradual warming to 60°C and stirring for 2 h. The reaction was monitored by TLC until the completion of the reaction. Then 5 mL of water was added to the reaction solution, and a white solid precipitated. Upon filtration and removal of water by rotary evaporation, the title compound 4-amino-2-fluoro-5-methylbenzamide **Intermediate 1** was obtained (white solid, 160 mg, yield 47%).

¹H NMR (400 MHz DMSO) δ 7.37 (d, 1H), 7.15 (s, 1H), 6.96 (s, 1H), 6.32 (d, 1H), 5.70 (s, 1H), 2.01 (s, 3H).

LC-MS m/z (ESI) = 169.10 [M+1].

### Intermediate 2

### 2-chloro-9-(tetrahydro-2H-pyran-4-yl)-7,9-dihydro-8H-purin-8-one (Intermediate 2)

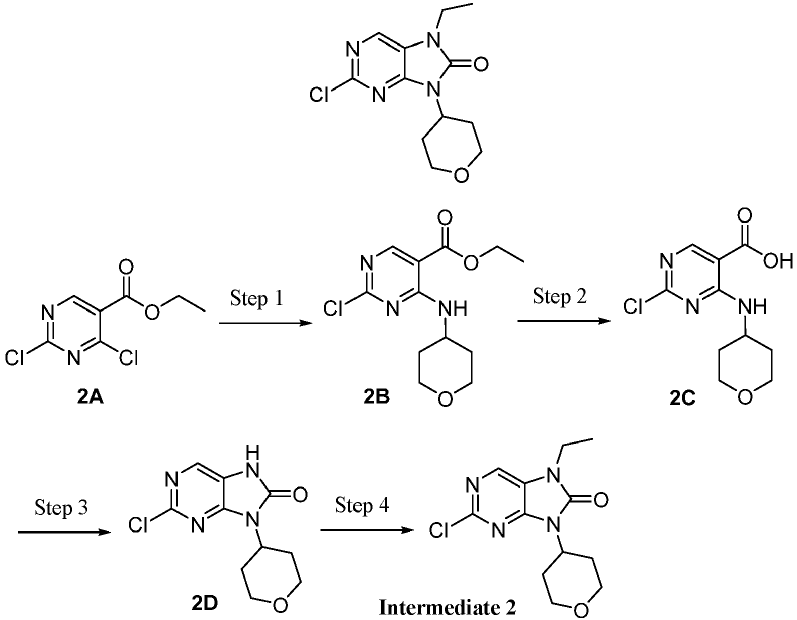

### Step 1:

### Ethyl 2-chloro-4-((tetrahydro-2H-pyran-4-yl)amino)pyrimidine-5-carboxylate (2B)

Ethyl 2,4-dichloropyrimidine-5-carboxylate **2A** (30.00 g, 136.4 mmol) and tetrahydro-2H-pyran-4-amine hydrochloride (18.66 g, 136.4 mmol) were dissolved in acetonitrile (600 mL). After stirring several times, potassium carbonate (46.92 g, 340.9 mmol) was added and stirred for 4 h at room temperature. After completion of the reaction as monitored by TLC, the reaction solution was filtered and the filter residue was washed with ethyl acetate (300 mL). The filtrate was concentrated to obtain a crude product which was purified by column separation (n-hexane: ethyl acetate (v/v)=1:1) to give the title compound ethyl 2-chloro-4-((tetrahydro-2H-pyran-4-yl)amino)pyrimidine-5-carboxylate **2B** (white solid, 30.0 g, yield 77.4%).

¹H NMR (400 MHz DMSO) δ 8.62 (s,1H), 8.32 (d, 1H), 4.30 (q, 2H), 4.21-4.16 (m, 1H), 3.86-3.83 (m, 2H), 3.48-3.42 (m, 2H), 1.88-1.85 (m, 2H), 1.62-1.53 (m, 2H), 1.31 (t, 3H).

LC-MS m/z (ESI) = 286.10 [M+1].

### Step 2:

### 2-chloro-4-((tetrahydro-2H-pyran-4-yl)amino)pyrimidine-5-carboxylic acid (2C)

Ethyl 2-chloro-4-((tetrahydro-2H-pyran-4-yl)amino)pyrimidine-5-carboxylate **2B** (30 g, 104.99 mmol) was dissolved in tetrahydrofuran/water (200 mL/200 mL), and lithium hydroxide (5.03 g, 209.99 mmol) was added thereto and stirred at room temperature for 1 h. After completion of the reaction as monitored by TLC, the reaction solution was concentrated to remove tetrahydrofuran, adjusted to pH 5 with 6N hydrochloric acid, and a white solid was precipitated. After filtration, the filter cake was washed twice with petroleum ether, and the solid was collected to give the title compound 2-chloro-4-((tetrahydro-2H-pyran-4-yl)amino)pyrimidine-5-carboxylic acid **2C** (white solid, 15.0 mg, yield 55.44%).

¹H NMR (400 MHz DMSO) δ 8.60 (s, 1H), 8.54 (d, 1H), 4.20-4.15 (m, 1H), 3.86-3.83 (m, 2H), 3.48-3.42 (m, 2H), 1.89-1.86 (m, 2H), 1.60-1.50 (m, 2H).

LC-MS m/z(ESI)= 258.10 [M+1].

### Step 3:

### 2-chloro-9-(tetrahydro-2H-pyran-4-yl)-7,9-dihydro-8H-purin-8-one (2D)

2-chloro-4-((tetrahydro-2H-pyran-4-yl)amino)pyrimidine-5-carboxylic acid **2C** (15 g, 58.21 mmol) was dissolved in dimethylacetamide (150 mL), and triethylamine (7.38 mL, 58.21 mmol) and diphenyl azidophosphate (12.06 mL, 58.21 mmol) were added thereto. The mixture was gradually warmed to 120°C, and reacted under stirring for 1.5 h. After completion of the reaction as monitored by TLC, the reaction solution was poured into ice water. The solid collected upon filtration was washed three times with water, and concentrated and dried under vacuum to obtain the title compound 2-chloro-9-(tetrahydro-2H-pyran-4-yl)-7,9- dihydro-8H-purin-8-one **2D** (white solid, 13.0 mg, yield 87.69%).

¹H NMR (400 MHz DMSO) δ 11.63 (s, 1H), 8.11 (s, 1H), 4.43-4.37 (m, 1H), 3.98-3.94 (m, 2H), 2.59-2.38 (m, 2H), 1.73-1.65 (m, 2H).

LC-MS m/z (ESI) = 255.10 [M+1].

### Step 4:

### 2-chloro-7-ethyl-9-(tetrahydro-2H-pyran-4-yl)-7,9-dihydro-8H-purin-8-one

### (Intermediate 2)

2-chloro-9-(tetrahydro-2H-pyran-4-yl)-7,9-dihydro-8H-purin-8-one **2D** (400 mg, 1.57 mmol) was dissolved in N,N-dimethylformamide (8 mL), cesium carbonate (511 mg, 1.57 mmol) and ethyl iodide (293 mg, 1.88 mmol) were added thereto at 0°C, and reacted under stirring for 1 h. The reaction was monitored by TLC until the completion of the reaction, followed by addition of 10 mL of water, and extracted three times with ethyl acetate. The organic phase was dried with anhydrous sodium sulfate, concentrated, and rotary evaporated to remove the organic solvent, and the title compound 2-chloro-7-ethyl-9-(tetrahydro-2H-pyran-4-yl)-7,9-dihydro-8H-purin-8-one **Intermediate 2** was obtained (white solid, 290 mg, yield 65.32%).

¹H NMR (400 MHz, DMSO-d6) δ 8.44 (s, 1H), 4.50-4.41 (m, 1H), 3.99-3.95 (m, 2H), 3.89 (q, 2H), 3.45 (t, 2H), 2.46-2.41 (m, 2H), 1.71-1.67 (m, 2H), 1.25 (t, 3H).

LC-MS m/z (ESI) = 283.10 [M+1].

### Example 1. Preparation of Compound A

### 4-((7-ethyl-8-oxo-9-(tetrahydro-2H-pyran-4-yl)-8,9-dihydro-7H-purin-2-yl)amino)- 2-fluoro-5-methylbenzamide (Compound A)

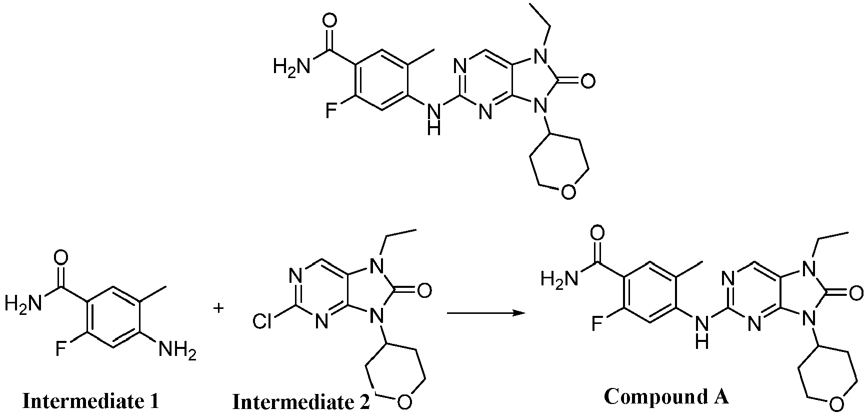

4-amino-2-fluoro-5-methylbenzamide (**Intermediate 1**) (350 mg, 2.12 mmol), 2-chloro-7-ethyl-9-(tetrahydro-2H-pyran-4-yl)-7,9-dihydro-8H-purin-8-one (**Intermediate 2**) (150 mg, 0.53 mmol), cesium carbonate (690 mg, 2.12 mmol), and methanesulfonato(2-dicyclohexylphosphino-3,6-dimethoxy-2',4',6'-triisopropyl-1,1'-biphenyl)(2'-amino-1,1'-biphenyl-2-yl)palladium(II) (72 mg, 0.08 mmol) were dissolved in 1,4-dioxane (5 mL), purged with nitrogen for protection, and reacted under stirring at 110°C for 4 h. The reaction was monitored by TLC until the completion of the reaction. The reaction solution was concentrated, and purified by silica gel column chromatography (dichloromethane/methanol (v/v)=20/1) and then subjected to Pre-HPLC, to give the title compound 4-((7-ethyl-8-oxo-9-(tetrahydro-2H-pyran-4-yl)-8,9-dihydro-7H-purin-2-yl)amino)-2-fluoro-5-methylbenzamide (**Compound A**) (white solid, 38 mg, yield 17.28%).

¹H NMR (400 MHz, DMSO-d6) δ 8.53 (s, 1H), 8.25 (s, 1H), 7.89 (d, 1H), 7.55 (d, 1H), 7.42 (d, 2H), 4.48-4.40 (m, 1H), 3.98 (dd, 2H), 3.85 (q, 2H), 3.43 (t, 2H), 2.58-2.54 (m, 2H), 2.30 (s, 3H) 1.71-1.68 (m, 2H), 1.25 (t, 3H).

LC-MS m/z(ESI)= 415.20 [M+1].

### Example 2. Preparation of crystalline form I of Compound A

530 g of the solid of Compound A was transferred to a reaction kettle and 15.9 L of a mixed solution (ethanol: water = 12.7 L: 3.2 L) was added. The mixed suspension was heated to reflux (reflux started at 72°C with gradual dissolution of the solid, and remained stable after heating to 76°C with dissolution of the solid into a yellow solution). After the solution became clear, it was refluxed and stirred for 1 h. The solution was kept overnight, and crystals slowly precipitated. After filtration, the filter cake was dried at 60°C for 16 h and then pulverized in a pulverizer. Subsequently, the solid was blow dried again at 60°C for 16 h.

### Example 3. Preparation of crystalline form I of Compound A

1.050 kg of Compound A, 23.0 kg of ethanol, and 9.3 kg of purified water were added sequentially into a reaction kettle. The reaction solution was heated up to 80±5°C for dissolution for 1 h and then cooled down to 65°C. The reaction solution was kept at 60±5°C for 1 h for crystallization, and then cooled to 25°C and kept at 20±5°C for 2 h for further crystallization. After filtration, the product was dried at 60±5°C for 16 h.

### Example 4. Preparation of crystalline form II of Compound A

0.1 kg of Compound A, 2.3 kg of ethanol, and 0.9 kg of purified water were added sequentially into a reaction kettle. The mixture solution was heated up to 80±5°C for dissolution for 1 h, and then cooled down to 60±5°C and kept for 1 h. The temperature was then lowered to 20±5°C and kept for 2 h. The resultant was filtered and dried at 60±5°C. Subsequently, the product was added to 1,4-dioxane, stirred at 50°C for 6 days, and vacuum dried at room temperature for 1 day to obtain a solid.

### Example 5. Preparation of crystalline form III of Compound A

0.1 kg of Compound A, 2.3 kg of ethanol, and 0.9 kg of purified water were added sequentially into a reaction kettle. The mixture solution was heated up to 80±5°C for dissolution for 1 h, and then cooled down to 60±5°C and kept for 1 h. The temperature was then lowered to 20±5°C and kept for 2 h. The resultant was filtered and dried at 60±5°C. Subsequently, the product was added to 1,4-dioxane, suspended and stirred at 50°C for 4 days, and vacuum dried at room temperature for 2 h to obtain a solid.

### Example 6. Preparation of crystalline form IV of Compound A

0.1 kg of Compound A, 2.3 kg of ethanol, and 0.9 kg of purified water were added sequentially into a reaction kettle. The reaction solution was heated up to 80±5°C for dissolution for 1 h, and then cooled down to 60±5°C and kept for 1 h. The temperature was lowered to 20±5°C and kept for 2 h. The resultant was filtered and dried at 60±5°C. Subsequently, the product was added to acetone, suspended and stirred at room temperature for 6 days, and left to dry at room temperature to obtain a solid.

### Example 7. Preparation of crystalline form V of Compound A

0.1 kg of Compound A, 2.3 kg of ethanol, and 0.9 kg of purified water were added sequentially into a reaction kettle. The mixture solution was heated up to 80±5°C for dissolution for 1 h, and then cooled down to 60±5°C and kept for 1 h. The temperature was then lowered to 20±5°C and kept for 2 h. The resultant was filtered and dried at 60±5°C. Subsequently, the product was added to 1,4-dioxane, and suspended and stirred at 50°C for 4 days. After vacuum drying at room temperature, the resulting solid was heated to 125°C and cooled to room temperature.

### Example 8. Preparation of crystalline form VI of Compound A

0.1 kg of Compound A, 2.3 kg of ethanol, and 0.9 kg of purified water were added sequentially into a reaction kettle. The mixture solution was heated up to 80±5°C for dissolution for 1 h, and then cooled down to 60±5°C and kept for 1 h. The temperature was then lowered to 20±5°C and kept for 2 h. The resultant was filtered and dried at 60±5°C. Subsequently, the product was added to acetone, and suspended and stirred at room temperature for 6 days. After drying at room temperature, the resulting solid was heated to 130°C and cooled to room temperature.

### Testing Example 1

The crystalline form I of compound A prepared in Example 2 was irradiated with graphite monochromatic Cu Kα radiation (λ = 1.54) at room temperature using an X-ray diffractometer from Dandong Haoyuan Instrument Co. Ltd. to obtain a powder diffraction pattern, and the data were analyzed.

The X-ray powder diffraction data of the crystalline form I of Compound A prepared in Example 2 are shown in Table 1.

**Table 1. X-ray powder diffraction data of crystalline form I of Compound A prepared in Example 2**

| **Peak number** | **2θ[°]** | **Crystal plane spacing** | **Peak height** | **Half peak width** | **Relative peak height** |
|---|---|---|---|---|---|
| 1 | 4.979 | 17.7328 | 897 | 0.202 | 23.1 |
| 2 | 9.859 | 8.9645 | 3883 | 0.177 | 100 |
| 3 | 11.483 | 7.7 | 221 | 0.178 | 5.7 |
| 4 | 12.12 | 7.2966 | 412 | 0.159 | 10.6 |
| 5 | 14.759 | 5.9972 | 1126 | 0.16 | 29 |
| 6 | 15.759 | 5.6189 | 785 | 0.152 | 20.2 |
| 7 | 16.601 | 5.3359 | 130 | 0.153 | 3.4 |
| 8 | 16.998 | 5.212 | 73 | 0.187 | 1.9 |
| 9 | 17.523 | 5.057 | 88 | 0.119 | 2.3 |
| 10 | 18.404 | 4.817 | 191 | 0.214 | 4.9 |
| 11 | 18.802 | 4.7158 | 567 | 0.169 | 14.6 |
| 12 | 19.339 | 4.5861 | 856 | 0.192 | 22 |
| 13 | 19.679 | 4.5076 | 1250 | 0.182 | 32.2 |
| 14 | 19.961 | 4.4445 | 1112 | 0.167 | 28.6 |
| 15 | 20.422 | 4.3453 | 212 | 0.156 | 5.5 |
| 16 | 21.379 | 4.1529 | 234 | 0.265 | 6 |
| 17 | 21.822 | 4.0696 | 646 | 0.227 | 16.6 |
| 18 | 22.142 | 4.0114 | 224 | 0.232 | 5.8 |
| 19 | 22.481 | 3.9516 | 902 | 0.164 | 23.2 |
| 20 | 22.877 | 3.8842 | 176 | 0.188 | 4.5 |
| 21 | 24.198 | 3.675 | 110 | 0.146 | 2.8 |
| 22 | 24.641 | 3.6099 | 946 | 0.228 | 24.4 |
| 23 | 25.939 | 3.4322 | 333 | 0.142 | 8.6 |
| 24 | 26.681 | 3.3384 | 128 | 0.177 | 3.3 |
| 25 | 26.961 | 3.3044 | 85 | 0.134 | 2.2 |
| 26 | 27.601 | 3.2292 | 100 | 0.215 | 2.6 |
| 27 | 28.038 | 3.1798 | 71 | 0.201 | 1.8 |
| 28 | 28.519 | 3.1273 | 81 | 0.146 | 2.1 |
| 29 | 29.18 | 3.0579 | 241 | 0.155 | 6.2 |
| 30 | 29.624 | 3.0131 | 148 | 0.22 | 3.8 |
| 31 | 30.077 | 2.9688 | 38 | 0.281 | 1 |
| 32 | 30.322 | 2.9453 | 61 | 0.275 | 1.6 |
| 33 | 30.619 | 2.9174 | 78 | 0.295 | 2 |
| 34 | 31.041 | 2.8787 | 360 | 0.201 | 9.3 |
| 35 | 31.723 | 2.8184 | 116 | 0.238 | 3 |
| 36 | 32.524 | 2.7508 | 66 | 0.256 | 1.7 |
| 37 | 33.379 | 2.6823 | 71 | 0.177 | 1.8 |
| 38 | 34.039 | 2.6317 | 84 | 0.284 | 2.2 |
| 39 | 35.001 | 2.5616 | 64 | 0.118 | 1.6 |
| 40 | 35.587 | 2.5207 | 103 | 0.269 | 2.7 |
| 41 | 37.178 | 2.4164 | 42 | 0.206 | 1.1 |
| 42 | 37.724 | 2.3827 | 73 | 0.138 | 1.9 |
| 43 | 38.319 | 2.347 | 51 | 0.307 | 1.3 |
| 44 | 38.61 | 2.33 | 46 | 0.195 | 1.2 |
| 45 | 39.125 | 2.3005 | 112 | 0.154 | 2.9 |

The X-ray powder diffraction pattern of the crystalline form I of Compound A prepared in Example 2 is shown in Fig. 1.

### Testing Example 2

XRPD results were collected on X'Pert3 and Empyrean X-ray powder diffraction analyzers for crystalline form I of Compound A prepared in Example 3, and the scanning parameters are shown in Table 2.

**Table 2. XRPD test parameters**

| **Mode** | **Conventional reflection** | **Variable temperature XRPD** |
|---|---|---|
| X-ray | Cu, ka; Kα1 (Å): 1.540598, Ka2 (Å): 1.544426 | |
| | Intensity ratio Kα2/Kα1: 0.50 | |
| X-Ray Tube Settings | 45 kV, 40 mA | 45 kV, 40 mA |
| Divergence slit | 1/8° | 1/8° |
| Scanning Mode | Continuous | Continuous |
| Scanning range (°2TH) | 3° ∼ 40° | 3° ∼ 40° |
| Scanning step (°2TH) | 0.0263 | 0.0167 |
| Scanning time per step (s) | 46.67 | 33.02 |
| Scanning time (s) | 5 min 04 s | 10 min 14 s |

The X-ray powder diffraction data of the crystalline form I of Compound A prepared in Example 3 are shown in Table 3.

**Table 3. X-ray powder diffraction data of crystalline form I of Compound A prepared in Example 3**

| **Peak number** | **2θ[°]** | **Crystal plane spacing [Å]** | **Relative peak height [%]** |
|---|---|---|---|
| 1 | 4.93 | 17.92 | 26.40 |
| 2 | 9.80 | 9.02 | 100.00 |
| 3 | 11.46 | 7.72 | 1.11 |
| 4 | 12.08 | 7.33 | 2.23 |
| 5 | 14.70 | 6.02 | 23.34 |
| 6 | 15.73 | 5.64 | 4.63 |
| 7 | 16.63 | 5.33 | 0.39 |
| 8 | 18.43 | 4.81 | 0.44 |
| 9 | 18.80 | 4.72 | 1.82 |
| 10 | 19.30 | 4.60 | 2.49 |
| 11 | 19.63 | 4.52 | 21.61 |
| 12 | 19.94 | 4.45 | 7.25 |
| 13 | 20.45 | 4.34 | 0.56 |
| 14 | 21.36 | 4.16 | 0.55 |
| 15 | 21.86 | 4.07 | 2.40 |
| 16 | 22.47 | 3.96 | 2.80 |
| 17 | 22.89 | 3.89 | 0.79 |
| 18 | 24.60 | 3.62 | 11.12 |
| 19 | 25.90 | 3.44 | 0.78 |
| 20 | 26.81 | 3.33 | 0.23 |
| 21 | 27.63 | 3.23 | 0.37 |
| 22 | 29.14 | 3.06 | 1.70 |
| 23 | 29.61 | 3.02 | 1.71 |
| 24 | 31.01 | 2.88 | 0.87 |
| 25 | 31.73 | 2.82 | 0.42 |
| 26 | 33.35 | 2.69 | 0.28 |
| 27 | 34.00 | 2.64 | 0.52 |
| 28 | 35.03 | 2.56 | 0.20 |
| 29 | 35.65 | 2.52 | 0.30 |
| 30 | 37.73 | 2.38 | 0.28 |
| 31 | 38.93 | 2.31 | 0.32 |
| 32 | 39.20 | 2.30 | 0.10 |
| 33 | 39.81 | 2.26 | 1.55 |

The X-ray powder diffraction pattern of the crystalline form I of Compound A prepared in Example 3 is shown in Fig. 2.

### Testing Example 3

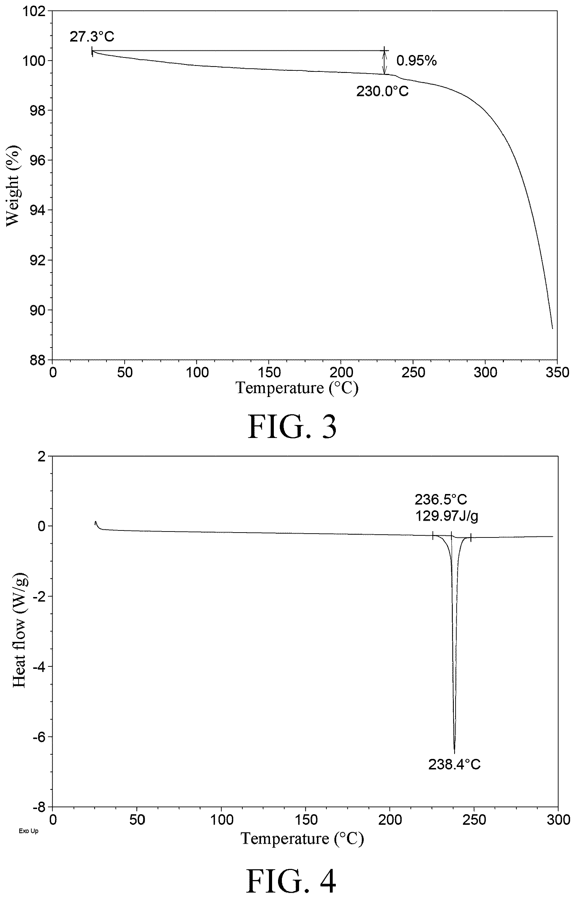

The crystalline form I of Compound A prepared in Example 3 was subjected to thermogravimetric analysis (TGA) and differential scanning calorimetry (DSC), respectively, and the TGA and DSC data were collected on a TA Q5000/Discovery 5500 thermogravimetric analyzer and a TA Discovery 2500 differential scanning calorimeter, respectively. TGA and DSC test parameters are listed in Table 4. The test results are shown in Figs. 3 and 4, respectively. TGA diagram showed a weight loss of 0.95% when heated to 230.0°C; the DSC diagram showed a peak endothermic peak at 238.4°C.

**Table 4. TGA and DSC test parameters**

| **Parameters** | **TGA** | **DSC** |
|---|---|---|
| Method | linear temperature rise | linear temperature rise |
| Sample disc | aluminum disc, open | aluminum disc, covered |
| Temperature range | room temperature to 350°C | 25°C to 300°C |
| Heating rate | 10°C/min | 10°C/min |
| Protective gas | nitrogen | nitrogen |

### Testing Example 4

The crystalline form I of Compound A prepared in Example 3 was subjected to dynamic vapor sorption test, and the dynamic vapor sorption (DVS) curves were acquired on DVS Intrinsic of SMS (Surface Measurement Systems). The relative humidity at 25°C was corrected with the deliquescence points of LiCl, Mg(NO₃)₂, and KCl. The DVS test parameters are listed in Table 5, and the test results are shown in Fig. 5. As can be seen from Fig. 5, the change in sample weight of the crystalline form I of Compound A of the present invention was less than 0.2% over the course of 0%RH-95%RH-0%RH at 25°C, indicating that the sample was free or almost free of hygroscopicity. Comparison of the X-ray powder diffraction patterns before and after the DVS showed that no transformation of crystalline form occurred before and after the DVS.

**Table 5. DVS test parameters**

| **Parameters** | **DVS** |
|---|---|
| Temperature | 25°C |
| Sample amount | 20 to 40 mg |
| Protective gas and flow rate | N₂, 200 mL/min |
| dm/dt | 0.002%/min |
| Minimum dm/dt equilibration time | 10 min |
| Maximum equilibration time | 180 min |
| RH range | 0%RH-95%RH-0%RH |
| RH gradient | 10%RH (0%RH-90%RH & 90%RH-0%RH) |
| | 5%RH (90%RH-95%RH & 95%RH-90%RH) |

### Testing Example 5

The XRPD result acquisition for crystalline form II was performed using the test conditions of Test Example 2. The X-ray powder diffraction data of crystalline form II are shown in Table 6.

**Table 6. X-ray powder diffraction data of crystalline form II**

| **Peak number** | **2θ[°]** | **Crystal plane spacing [Å]** | **Relative peak height [%]** |
|---|---|---|---|
| 1 | 4.76 | 18.55 | 23.25 |
| 2 | 8.61 | 10.27 | 22.56 |
| 3 | 9.52 | 9.29 | 55.56 |
| 4 | 10.46 | 8.46 | 100.00 |
| 5 | 13.78 | 6.42 | 98.29 |
| 6 | 14.29 | 6.20 | 32.58 |
| 7 | 15.51 | 5.71 | 2.05 |
| 8 | 17.18 | 5.16 | 13.34 |
| 9 | 18.39 | 4.82 | 4.04 |
| 10 | 18.60 | 4.77 | 6.55 |
| 11 | 19.08 | 4.65 | 23.26 |
| 12 | 19.75 | 4.50 | 1.77 |
| 13 | 23.90 | 3.72 | 4.91 |
| 14 | 24.12 | 3.69 | 6.35 |
| 15 | 24.93 | 3.57 | 1.97 |
| 16 | 25.97 | 3.43 | 9.34 |
| 17 | 26.29 | 3.39 | 6.10 |
| 18 | 27.77 | 3.21 | 1.31 |
| 19 | 29.78 | 3.00 | 1.68 |
| 20 | 30.87 | 2.90 | 2.67 |
| 21 | 34.77 | 2.58 | 3.79 |
| 22 | 36.00 | 2.49 | 2.65 |
| 23 | 37.69 | 2.39 | 1.34 |
| 24 | 38.38 | 2.35 | 0.96 |

The X-ray powder diffraction pattern of crystalline form II is shown in Fig. 6.

### Testing Example 6

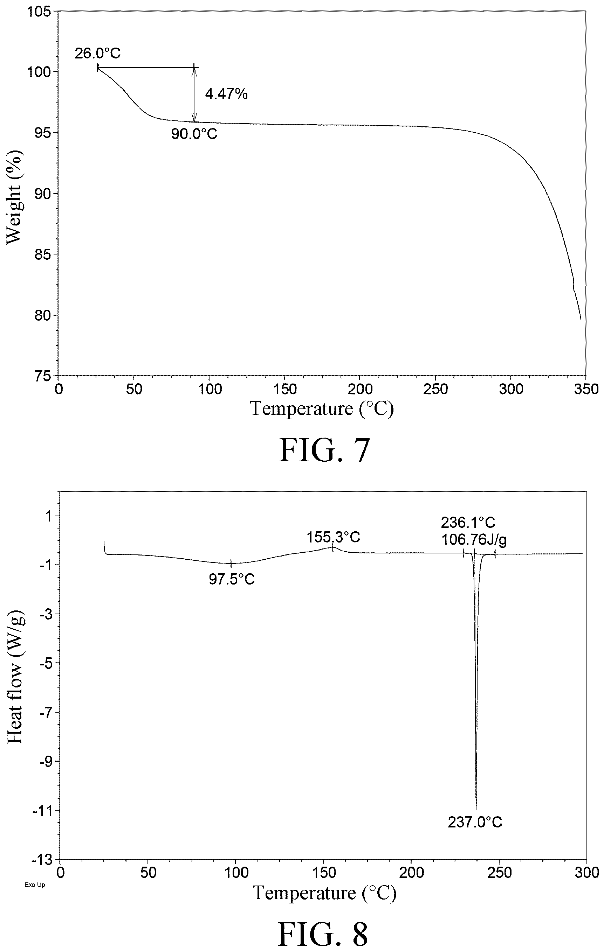

Thermogravimetric analysis (TGA) and differential scanning calorimetry (DSC) of crystalline type II were performed using the test conditions of Test Example 3, and the test results are shown in Figs. 7 and 8, respectively. The TGA diagram shows a step-like weight loss of 4.47% when heated to 90°C; the DSC diagram shows two endothermic peaks at 97.5°C (peak temperature) and 236.1°C (onset temperature), and one exothermic peak at 155.3°C (peak temperature).

### Testing Example 7

The acquisition of the XRPD results of crystalline form III was performed using the test conditions of Test Example 2. The X-ray powder diffraction data of crystalline form III are shown in Table 7.

**Table 7. X-ray powder diffraction data of crystalline form III**

| **Peak number** | **2θ[°]** | **Crystal plane spacing [Å]** | **Relative peak height [%]** |
|---|---|---|---|
| 1 | 8.49 | 10.42 | 100.00 |
| 2 | 9.02 | 9.81 | 20.62 |
| 3 | 9.27 | 9.54 | 40.05 |
| 4 | 13.49 | 6.56 | 5.34 |
| 5 | 15.32 | 5.78 | 28.54 |
| 6 | 15.78 | 5.61 | 15.30 |
| 7 | 17.82 | 4.98 | 34.66 |
| 8 | 18.07 | 4.91 | 65.14 |
| 9 | 18.28 | 4.85 | 20.65 |
| 10 | 18.56 | 4.78 | 11.45 |
| 11 | 18.99 | 4.67 | 22.46 |
| 12 | 19.97 | 4.45 | 5.15 |
| 13 | 20.96 | 4.24 | 9.50 |
| 14 | 22.54 | 3.94 | 9.32 |
| 15 | 23.04 | 3.86 | 14.02 |
| 16 | 23.45 | 3.79 | 87.65 |
| 17 | 24.21 | 3.68 | 19.98 |
| 18 | 24.65 | 3.61 | 16.11 |
| 19 | 25.61 | 3.48 | 13.80 |
| 20 | 26.26 | 3.39 | 22.82 |
| 21 | 28.16 | 3.17 | 3.62 |
| 22 | 29.14 | 3.06 | 26.27 |
| 23 | 30.18 | 2.96 | 12.97 |
| 24 | 35.01 | 2.56 | 3.39 |

The X-ray powder diffraction pattern of crystalline form III is shown in Fig. 9.

### Testing Example 8

Thermogravimetric analysis (TGA) and differential scanning calorimetry (DSC) of crystalline type III were performed using the test conditions of Test Example 3, and the test results are shown in Figs. 10 and 11, respectively. The TGA diagram shows a weight loss of 2.5% when heated to 100°C, and a step-like loss of 17.7% with continued heating to 150°C. The DSC diagram shows one endothermic signal at 112.1°C (onset temperature), presumably due to dehydration or solvent, and two endothermic peaks at 234.7°C and 236.7°C (peak temperature).

### Testing Example 9

The acquisition of the XRPD results of crystalline form IV was performed using the test conditions of Test Example 2. The X-ray powder diffraction data of crystalline form IV are shown in Table 8.

**Table 8. X-ray powder diffraction data of crystalline form IV**

| **Peak number** | **2θ[°]** | **Crystal plane spacing [Å]** | **Relative peak height [%]** |
|---|---|---|---|
| 1 | 6.59 | 13.41 | 2.12 |
| 2 | 9.39 | 9.42 | 12.84 |
| 3 | 10.55 | 8.39 | 100.00 |
| 4 | 11.18 | 7.92 | 6.60 |
| 5 | 13.92 | 6.36 | 4.05 |
| 6 | 14.41 | 6.15 | 14.89 |
| 7 | 14.60 | 6.07 | 11.19 |
| 8 | 15.67 | 5.65 | 0.97 |
| 9 | 17.36 | 5.11 | 2.20 |
| 10 | 18.50 | 4.80 | 12.05 |
| 11 | 18.89 | 4.70 | 8.68 |
| 12 | 19.73 | 4.50 | 5.13 |
| 13 | 20.45 | 4.34 | 3.77 |
| 14 | 21.15 | 4.20 | 5.47 |
| 15 | 22.28 | 3.99 | 69.06 |
| 16 | 24.34 | 3.66 | 8.19 |
| 17 | 24.69 | 3.61 | 5.44 |
| 18 | 25.69 | 3.47 | 1.49 |
| 19 | 26.41 | 3.38 | 2.71 |
| 20 | 26.93 | 3.31 | 8.52 |
| 21 | 28.88 | 3.09 | 24.98 |
| 22 | 29.54 | 3.02 | 2.40 |
| 23 | 31.96 | 2.80 | 1.48 |
| 24 | 33.93 | 2.64 | 0.74 |
| 25 | 34.91 | 2.57 | 0.88 |
| 26 | 37.57 | 2.39 | 1.16 |

The X-ray powder diffraction pattern of crystalline form VI is shown in Fig. 12.

### Testing Example 10

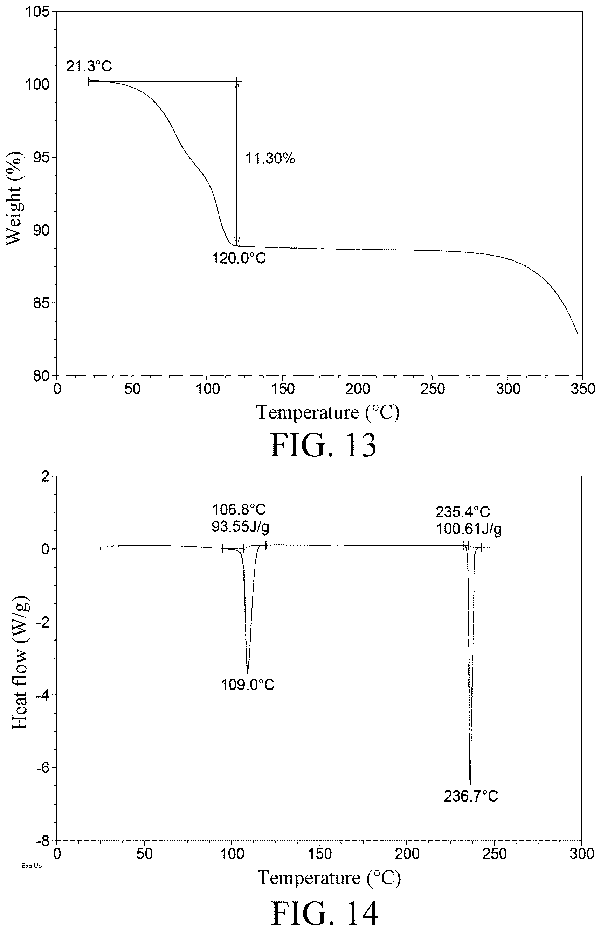

Thermogravimetric analysis (TGA) and differential scanning calorimetry (DSC) of crystalline type IV were performed using the test conditions of Test Example 3, and the test results are shown in Figs. 13 and 14, respectively. The TGA diagram shows a step-like weight loss of 11.3% when heated to 120°C; the DSC diagram shows two endothermic peaks at 106.8°C and 235.4°C (onset temperature).

### Testing Example 11

The acquisition of the XRPD results of crystalline form V was performed using the test conditions of Test Example 2. The X-ray powder diffraction data of crystalline form V are shown in Table 9.

**Table 9. X-ray powder diffraction data of crystalline form V**

| **Peak number** | **2θ[°]** | **Crystal plane spacing [Å]** | **Relative peak height [%]** |
|---|---|---|---|
| 1 | 4.87 | 18.13 | 4.79 |
| 2 | 8.91 | 9.93 | 24.23 |
| 3 | 9.76 | 9.06 | 15.99 |
| 4 | 10.48 | 8.44 | 100.00 |
| 5 | 11.35 | 7.79 | 3.62 |
| 6 | 12.47 | 7.10 | 5.28 |
| 7 | 13.83 | 6.40 | 4.44 |
| 8 | 14.96 | 5.92 | 13.14 |
| 9 | 15.69 | 5.65 | 13.64 |
| 10 | 16.22 | 5.47 | 3.93 |
| 11 | 17.84 | 4.97 | 10.06 |
| 12 | 18.63 | 4.76 | 5.66 |
| 13 | 19.66 | 4.52 | 9.73 |
| 14 | 20.80 | 4.27 | 5.76 |
| 15 | 21.78 | 4.08 | 11.99 |
| 16 | 22.42 | 3.97 | 21.98 |
| 17 | 24.93 | 3.57 | 48.20 |
| 18 | 25.82 | 3.45 | 4.67 |
| 19 | 26.59 | 3.35 | 1.88 |
| 20 | 27.42 | 3.25 | 2.23 |
| 21 | 28.08 | 3.18 | 2.98 |
| 22 | 29.49 | 3.03 | 1.33 |
| 23 | 30.84 | 2.90 | 2.05 |
| 24 | 32.82 | 2.73 | 4.02 |
| 25 | 36.06 | 2.49 | 1.57 |

The X-ray powder diffraction pattern of crystalline form V is shown in Fig. 15.

### Testing Example 12

Thermogravimetric analysis (TGA) and differential scanning calorimetry (DSC) of crystalline type V were performed using the test conditions of Test Example 3, and the test results are shown in Figs. 16 and 17, respectively. The TGA diagram shows a weight loss of 1.8% when heated to 230°C; the DSC diagram shows two endothermic signals at 234.7°C and 236.7°C (peak temperature).

### Testing Example 13

The acquisition of the XRPD results of crystalline form VI was performed using the test conditions of Test Example 2. The X-ray powder diffraction data of crystalline form VI are shown in Table 10.

**Table 10. X-ray powder diffraction data of crystalline form VI**

| **Peak number** | **2θ[°]** | **Crystal plane spacing [Å]** | **Relative peak height [%]** |
|---|---|---|---|
| 1 | 10.66 | 8.30 | 2.32 |
| 2 | 11.52 | 7.68 | 100.00 |
| 3 | 14.44 | 6.13 | 9.59 |
| 4 | 14.96 | 5.92 | 8.51 |
| 5 | 15.44 | 5.74 | 2.95 |
| 6 | 16.39 | 5.41 | 10.92 |
| 7 | 18.85 | 4.71 | 2.78 |
| 8 | 19.35 | 4.59 | 3.12 |
| 9 | 20.78 | 4.28 | 1.53 |
| 10 | 21.45 | 4.14 | 4.31 |
| 11 | 22.12 | 4.02 | 4.58 |
| 12 | 22.41 | 3.97 | 5.15 |
| 13 | 23.39 | 3.80 | 49.07 |
| 14 | 24.60 | 3.62 | 2.36 |
| 15 | 25.27 | 3.52 | 3.41 |
| 16 | 30.15 | 2.96 | 16.10 |
| 17 | 33.19 | 2.70 | 1.73 |

The X-ray powder diffraction pattern of crystalline form VI is shown in Fig. 18.

### Testing Example 14

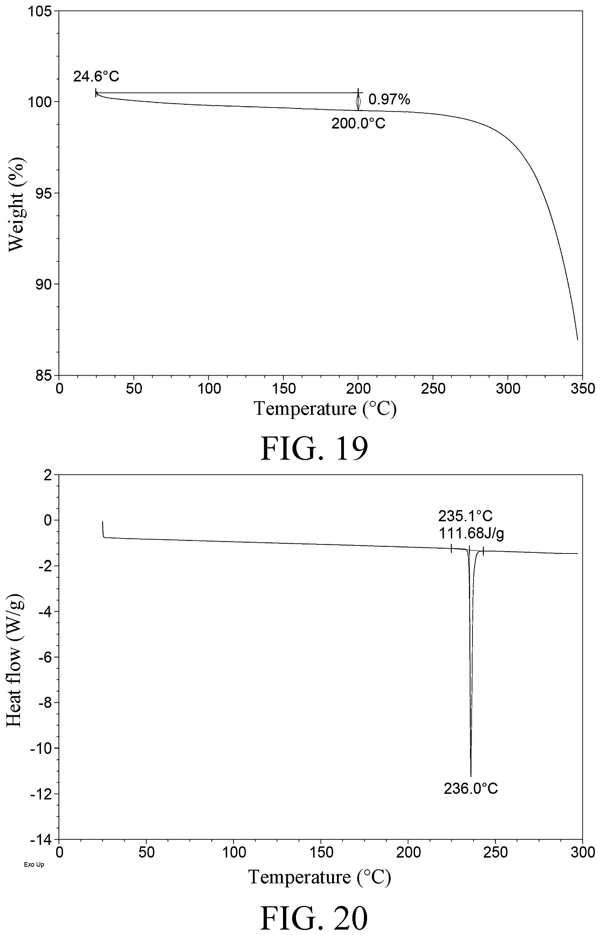

Thermogravimetric analysis (TGA) and differential scanning calorimetry (DSC) of crystalline type VI were performed using the test conditions of Test Example 3, and the test results are shown in Figs. 19 and 20, respectively. The TGA diagram shows a weight loss of 1.0% when heated to 200°C; the DSC diagram shows one endothermic peak at 235.1°C (onset temperature).

### Testing Example 15

After being kept under the conditions of 80°C/close and 60°C/close for 1 day, and under the conditions of 25°C/60%RH/open and 40°C/75%RH/open for 1 week, no crystalline transformation or decrease in purity was observed for crystalline form I (the physical stability of the crystalline form was assessed by XRPD test, and the chemical stability of the crystalline form was assessed by HPLC test for purity), which indicates that crystalline form I has a good stability in the solid state under the conditions assessed.

### Biological Test Examples

### 1. DNA-PK kinase inhibition assay

The inhibitory activity of the compound on a DNA-PK kinase was assayed by a DNA-PK kinase assay kit (purchased from Promega, Cat. No.: V4107, Batch No.: 0000366495). The results were quantified using chemiluminescence, with the following experimental protocol:
i. Creating a standard curve of ADP at different concentrations and fluorescence in accordance with the instructions for the kit;
ii. preparing 5 µL of a reaction system in a 384-well white plate, with 1 µL of the compound (with the concentration gradient set to 1 µM, 200 nM, 40 nM, 8 nM, 1.6 nM, 0.32 nM, 0.064 nM, 0.013 nM, respectively), 20 units of DNA-PK kinase, 0.2 µg/µL of a substrate, 10 µg/µL of DNA, 50 µM ATP, and 1% DMSO added in each well;
iii. mixing uniformly, centrifuging (1000 rpm, 30 s), and incubating at 37°C for 60 min;
iv. adding 5 µL of ADP-Glo^{™} Reagent to terminate the reaction, mixing uniformly, centrifuging (1000 rpm, 30 s), and incubating at room temperature for 40min;
v. adding 10 µL of Kinase Detection Reagent, mixing uniformly with shaking, centrifuging (1000 rpm, 30 s), and incubating at room temperature for 30 min;
vi. measuring the fluorescence using a microplate reader (Thermo fisher, Varioskan LUX). IC₅₀ was calculated using GraphPad Prism 8, and the results are shown in Table 11.

**Table 11. Inhibitory activity against DNA-PK kinase**

| Compound No. | IC₅₀ (nM) |
|---|---|
| Compound A | 4.17 |
| +Control example | 100.40 |

| | |
|---|---|
| Note: The control example is the Compound 3 in J. Med. Chem (2020), 63(7), 3461-3471, and was obtained according to the preparation method thereof. | |

The results show that the compound of the present invention has a more significant inhibitory effect on a DNA-PK kinase compared to the control example.

### 2. Pharmacokinetic assay

### 2.1 Test materials

ICR mice (purchased from Beijing Viton Lihua Laboratory Animal Technology Co.)

### 2.2 Experimental procedure

(1) Healthy male ICR mice (18-22g) were prepared, with 18 mice for each compound which were divided into 2 groups (iv and po groups) of 9 mice each, and 3 mice were selected for blood collection at each time point.
(2) After fasting (free water intake) overnight, the compound of the invention was solubilized (or formed into a suspension) with 5% DMSO, 95% 30% HP-β-CD (v:v) as solvent. The compound was administered via the tail vein (iv, 1 mg/kg) and by gavage (po, 10 mg/kg), respectively.
(3) In the iv group, 0.1 mL of blood was collected from the submandibular vein at 5 min, 15 min, 0.5 h, 1 h, 2 h, 4 h, 8 h, 12 h, and 24 h, respectively, and the blood was anticoagulated with EDTA-K2, and then centrifuged at 4°C for 5 min to separate the plasma, which was stored at -20°C before measurement.
(4) In the po group, 0.1 mL of blood was collected from the submandibular vein before and after administration at 15 min, 0.5h, 1h, 2h, 4h, 6h, 8h, 12h and 24h respectively, and processed in the same manner as in the iv group.
(5) The concentration of the compound of the present invention in plasma was determined by a LC/MS/MS method.
(6) The results were calculated and fitted using Analyst 1.6 from AB.

The test results show that Compound A and the crystalline forms I, II, III, IV, V, and VI has good pharmacokinetic properties.

The specific embodiments are described in detail in the specification of the present invention. Those skilled in the art would recognize that the above embodiments are illustrative and are not to be construed as a limitation of the present invention. For those skilled in the art, various improvement and modification may be made to the present invention without departing from the principles of the present invention, and the technical solutions obtained by such improvement and modification also fall within the protection scope of the claims of the present invention.

## Claims

1. A crystal of the compound represented by formula (A):

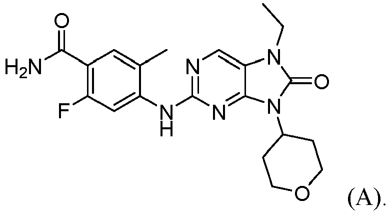

2. The crystal according to claim 1, wherein crystalline form I has characteristic diffraction peaks at 2θ positions of 9.859°±0.3°, 14.759°±0.3°, 19.679°±0.3°, and 19.961°±0.3° in an X-ray powder diffraction pattern obtained by Cu-Kα radiation.

3. The crystal according to claim 2, wherein the crystalline form I further has characteristic diffraction peaks at 2θ positions of 4.979°±0.2°, 15.759°±0.2°, 19.339°±0.2°, 22.481°±0.2°, and 24.641°±0.2° in the X-ray powder diffraction pattern obtained by Cu-Kα radiation.

4. The crystal according to claim 3, wherein the crystalline form I further has characteristic diffraction peaks at 2θ positions of 12.120°±0.2°, 18.802°±0.2°, and 21.822°±0.2° in the X-ray powder diffraction pattern obtained by Cu-Kα radiation.

5. The crystal according to claim 4, wherein the crystalline form I has an X-ray powder diffraction pattern substantially as shown in Fig. 1 or 2.

6. The crystal according to any one of claims 2 to 5, wherein the crystalline form I has a TGA curve substantially as shown in Fig. 3.

7. The crystal according to any one of claims 2 to 5, wherein the crystalline form I has a DSC curve substantially as shown in Fig. 4.

8. The crystal according to claim 1, wherein crystalline form II has an X-ray powder diffraction pattern substantially as shown in Fig. 6.

9. The crystal according to claim 1, wherein crystalline form III has an X-ray powder diffraction pattern substantially as shown in Fig. 9.

10. The crystal according to claim 1, wherein crystalline form IV has an X-ray powder diffraction pattern substantially as shown in Fig. 12.

11. The crystal according to claim 1, wherein crystalline form V has an X-ray powder diffraction pattern substantially as shown in Fig. 15.

12. The crystal according to claim 1, wherein crystalline form VI has an X-ray powder diffraction pattern substantially as shown in Fig. 18.

13. A method for preparing the crystalline form I defined in any one of claims 2 to 7, which is Method I or Method II:
Method I: dissolving Compound A in a solvent, heating to reflux till the solid is dissolved, and then naturally cooling down for crystallization, followed by filtering and drying, to obtain the crystalline form I;
Method II: dissolving Compound A in a solvent, heating to 75-85°C till the solid is dissolved, then cooling down to 55-65°C for crystallization, and further cooling down to 15-25°C for crystallization, followed by filtering and drying, to obtain the crystalline form I;
wherein the solvent is selected from an alcohol-based solvent, and a mixed solvent of an alcohol-based solvent and water.

14. A pharmaceutical composition comprising a therapeutically effective amount of the crystalline form according to any one of claims 1 to 12, and a pharmaceutically acceptable carrier or excipient.

15. Use of the crystalline form according to any one of claims 1 to 12 or the pharmaceutical composition according to claim 14 in the preparation of a DNA-PK inhibitor.

16. Use of the crystalline form according to any one of claims 1 to 12 or the pharmaceutical composition according to claim 14 in the preparation of a medicament for the treatment and prevention of cancer.
